(19) Europäisches Patentamt
European Patent Office
Office européen des brevets

(11) EP 0 742 002 B1

(12) **FASCICULE DE BREVET EUROPEEN**

(45) Date de publication et mention de la délivrance du brevet:
**22.10.1997 Bulletin 1997/43**

(51) Int. Cl.$^6$: **A61K 7/42**

(21) Numéro de dépôt: **96400551.6**

(22) Date de dépôt: **18.03.1996**

(54) **Compositions cosmétiques autobronzantes**

Selbstbräunende kosmetische Mittel

Artificial tanning compositions

(84) Etats contractants désignés:
**AT BE CH DE ES FR GB IT LI NL SE**

(30) Priorité: **07.04.1995 FR 9504192**

(43) Date de publication de la demande:
**13.11.1996 Bulletin 1996/46**

(73) Titulaire: **L'OREAL**
**75008 Paris (FR)**

(72) Inventeur: **Ascione, Jean-Marc**
**75018 Paris (FR)**

(74) Mandataire: **Andral, Christophe André Louis**
**L'OREAL**
**Centre de Recherche Charles Zviak**
**Département Propriété Industrielle**
**90, rue du Général Roguet**
**92583 Clichy Cedex (FR)**

(56) Documents cités:
**EP-A- 0 547 864**
**EP-A- 0 576 189**
**WO-A-94/22418**
**FR-A- 2 698 267**
**US-A- 5 232 688**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 742 002 B1

## Description

La présente invention concerne de nouvelles compositions cosmétiques à usage topique plus particulièrement destinées au bronzage et/ou au brunissage artificiel de la peau (compositions ci-après dénommées plus simplement compositions autobronzantes), ainsi que leur utilisation dans l'application cosmétique susmentionnée. Plus précisément encore, elle concerne des compositions autobronzantes à activité et stabilité améliorées se présentant sous la forme d'émulsions de type eau-dans-silicone particulières (support cosmétiquement acceptable) et comprenant, à titre d'agent d'autobronzage, de la dihydroxyacétone.

On sait que la dihydroxyacétone, ou DHA, est un produit particulièrement intéressant qui est couramment utilisé en cosmétique comme agent de bronzage artificiel de la peau ; appliqué sur cette dernière, notamment sur le visage, il permet d'obtenir un effet de bronzage ou de brunissage d'apparence semblable à celui qui peut résulter d'une exposition prolongée au soleil (bronzage naturel) ou sous une lampe UV. Une telle utilisation présente en outre pour avantage d'éviter totalement les risques de réaction cutanée généralement attachés aux expositions prolongées précitées (érythèmes, brûlures, perte d'élasticité, apparition de rides, vieillissement prématuré de la peau, et autres).

Pour diverses raisons liées en particulier à un meilleur confort d'utilisation (douceur, émollience, facilité d'application), les compositions autobronzantes actuelles se présentent le plus souvent sous la forme d'une émulsion de type huile-dans-eau (c'est à dire un support constitué d'une phase continue dispersante aqueuse et d'une phase discontinue dispersée huileuse) dans laquelle on a introduit, à des concentrations diverses, de la dihydroxyacétone qui, compte tenu de son caractère hydrophile, se retrouve présente dans la phase aqueuse de l'émulsion.

Il est connu des documents EP-A-0 576 189, US 5,232,688, EP-A-0 547 864, FR-A-2 698 267, WO-A-9 422 418 des compositions autobronzantes comprenant de la DHA en association avec divers composés tels que des silicones, des polyacrylamides, des aminoacides ou encore des émulsionnants particuliers.

Toutefois, l'un des inconvénients des compositions autobronzantes connues à ce jour et appartenant au type ci-dessus (émulsion H/E contenant de la DHA) est que l'intensité de la coloration obtenue sur la peau et/ou la rapidité avec laquelle cette coloration se développe, peuvent apparaître comme encore insuffisantes.

Par ailleurs, une autre difficulté réside dans le fait que la DHA présente une fâcheuse tendance, plus ou moins prononcée selon la nature du milieu dans lequel elle est formulée, à se dégrader au cours du temps, occasionnant par là des problèmes de stockage et/ou de conservation qui se traduisent généralement à terme par un jaunissement non souhaitable des compositions qui la contiennent.

La présente invention a notamment pour but de résoudre les problèmes ci-dessus en proposant de nouvelles émulsions à base de DHA qui présentent une efficacité et/ou une activité autobronzantes sur la peau améliorées (intensité et tenue), ainsi que, d'autre part, une excellente stabilité.

Ainsi, à la suite d'importantes recherches menées sur la question, il a maintenant été trouvé par la Demanderesse, et ceci de façon tout à fait inattendue et surprenante, qu'en formulant de la DHA dans des émulsions eau-dans-silicone contenant d'une part, un émulsionnant siliconé particulier, et d'autre part, un alcool mono ou dihydrique, on pouvait améliorer de manière substantielle la qualité de la coloration de la peau en termes d'intensité et d'homogénéité, ainsi que la stabilité de la DHA.

Cette découverte est à la base de la présente invention.

Ainsi, conformément à l'un des objets de la présente invention, il est maintenant proposé de nouvelles compositions cosmétiques destinées au bronzage artificiel de la peau, comprenant, dans un support cosmétiquement acceptable de type émulsion eau-dans-silicone, de la dihydroxyacétone à titre d'agent autobronzant, et qui sont essentiellement caractérisées par le fait qu'elles contiennent en outre:

- au moins un alcool mono ou dihydrique,
- au moins un émulsionnant siliconé constitué d'un polydiméthylsiloxane de formule (I) ou (II), éventuellement dispersé dans un polydiméthylsiloxane volatil,

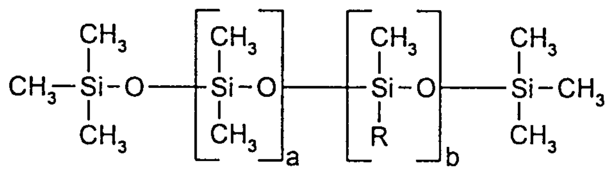

(I)

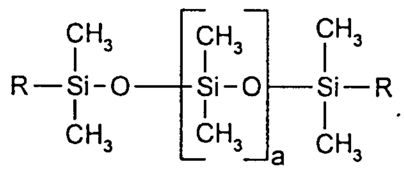

(II)

formules (I) et (II) dans lesquelles:

- $1 \leq a \leq 500$ (a étant un nombre entier)
- $1 \leq b \leq 100$ (b étant un nombre entier)
- R représente un radical:

$$—C_nH_{2n}—(—OC_2H_4)_x—(—OC_3H_6)_y—O—R'$$

dans lequel R' représente H ou un radical alkyle linéaire ou ramifié en $C_1$-$C_{12}$ et:

- $0 \leq x \leq 50$ (x étant un nombre entier)
- $0 \leq y \leq 50$ (y étant un nombre entier)
- $x + y \geq 1$
- $2 \leq n \leq 12$ (n étant un nombre entier)

Outre leurs propriétés autobronzantes intrinsèques améliorées, les compositions conformes à l'invention présentent une très bonne stabilité dans le temps. Elles présentent de plus l'avantage, très apprécié commercialement, de pouvoir être transparentes lorsque l'indice de réfraction i de la phase aqueuse est voisin de celui de la phase continue siliconée, à savoir lorsque :

$$| \text{ i phase siliconée - i phase aqueuse } | \leq 0{,}01.$$

D'autres caractéristiques, aspects et avantages de l'invention apparaîtront à la lecture de la description détaillée qui va suivre.

La dihydroxyacétone ou DHA est présente dans l'émulsion eau-dans-silicone selon l'invention dans des proportions suffisantes pour conférer à la peau, après application, une coloration similaire à la coloration obtenue à la suite d'un bronzage naturel. Elle est ainsi généralement présente dans des proportions comprises entre 0,5 et 10% en poids par rapport au poids total de l'émulsion, et de préférence comprises entre 1 et 7% en poids par rapport au poids total de la composition.

La ou les silicones constituant la phase continue de l'émulsion sont généralement des silicones volatiles, linéaires ou cycliques. De préférence, il s'agit de polydiméthylsiloxanes volatils, linéaires ou cycliques. De tels polydiméthylsiloxanes sont par exemple la cyclométhicone vendue sous la dénomination commerciale ⟨⟨ Silbione Huile 70 047 V 2 ⟩⟩ par Rhône-Poulenc.

Les silicones sont généralement présentes dans les compositions selon l'invention en une proportion comprise entre 1 et 50%, de préférence entre 5 et 25% en poids, par rapport au poids total de la composition.

Selon une caractéristiquè essentielle de la présente invention, les compositions conformes à l'invention comprennent également un troisième composé qui est un émulsionnant siliconé spécifique à poids moléculaire élevé constitué d'un polydiméthylsiloxane portant des chaînes polyoxyéthylènes et/ou polyoxypropylènes greffées sur la chaîne principale, cet émulsionnant présentant la structure (I) ou (II) suivante:

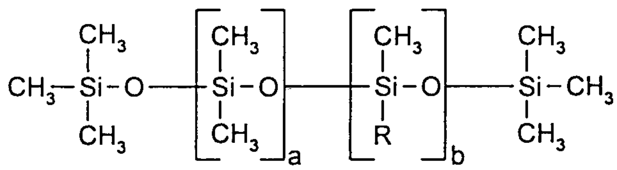

(I)

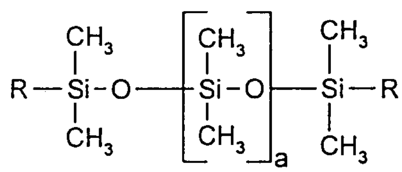

(II)

formules (I) et (II) dans lesquelles:

- $1 \leq a \leq 500$ (a étant un nombre entier)
- $1 \leq b \leq 100$ (b étant un nombre entier)
- R représente un radical:

$$-C_nH_{2n}-(-OC_2H_4)_x-(-OC_3H_6)_y-O-R'$$

dans lequel R' représente H ou un radical alkyle linéaire ou ramifié en $C_1$-$C_{12}$ et:

- $0 \leq x \leq 50$ (x étant un nombre entier)
- $0 \leq y \leq 50$ (y étant un nombre entier)
- $x + y \geq 1$
- $2 \leq n \leq 12$ (n étant un nombre entier)

De préférence, selon l'invention, on met en oeuvre des émulsionnants siliconés présentant au moins l'une, et encore plus préférentiellement l'ensemble, des caractéristiques suivantes:

- $2 \leq a \leq 450$
- $2 \leq b \leq 40$
- $1 \leq x \leq 30$
- $0 \leq y \leq 30$
- $y \leq x$

A titre d'exemples de radicaux R' alkyles linéaires ou ramifiés en $C_1$-$C_{12}$, on peut notamment citer les radicaux méthyle, éthyle, n-propyle, isopropyle, n-butyle, isobutyle, pentyle et hexyle.

De préférence, selon la présente invention, R' représente l'hydrogène.

Dans une forme préférée de réalisation de l'invention, l'émulsionnant siliconé est le polydiméthylsiloxane répondant à la formule suivante:

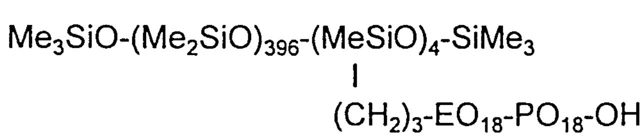

dans laquelle EO représente un motif d'oxyde d'éthylène et PO représente un motif d'oxyde de propylène.

A titre d'exemple d'émulsionnant siliconé convenant particulièrement bien pour la mise en oeuvre de la présente invention, on peut citer le polydiméthylsiloxane vendu sous la dénomination commerciale de ⟨⟨ Silicone

$Q_23225C$ 〉〉 par la société Dow Corning.

Le ou les émulsionnants siliconés sont généralement présents dans les compositions selon l'invention en une proportion comprise entre 0,1 et 20% en poids, de préférence entre 0,5 et 10% en poids, par rapport au poids total de la composition.

Selon une autre caractéristique essentielle de la présente invention, les compositions conformes à l'invention comprennent également au moins un alcool mono ou dihydrique, de préférence un alcool dihydrique.
A titre de mono-alcools utilisables selon l'invention, on peut citer l'éthanol, l'isopropanol.
A titre d'alcools dihydriques, on peut tout particulièrement citer l'isopentyldiol, le butylène glycol ou encore le propylène glycol. Selon un mode particulièrement préféré de réalisation de l'invention, on met en oeuvre, à titre d'alcool dihydrique, du propylène glycol.

De préférence, le ou les alcools mono ou dihydriques sont présents dans la composition en une proportion d'au moins 2% et encore plus préférentiellement en une proportion comprise entre 10 et 50% en poids par rapport au poids total de la composition.

Les compositions autobronzantes conformes à l'invention peuvent se présenter sous forme de crèmes, de laits, de gels, de gel-crèmes, de lotions fluides, en particulier de lotions fluides vaporisables, ou tout autre forme généralement utilisée en cosmétique, en particulier celle convenant habituellement aux compositions cosmétiques autobronzantes.

Comme indiqué précédemment, la dihydroxyacétone, compte tenu de son caractère hydrosoluble, est présente dans la phase aqueuse des émulsions selon l'invention.

Parmi les adjuvants cosmétiques classiques susceptibles d'être contenus dans la phase aqueuse et/ou dans la phase siliconée des émulsions conformes à l'invention (selon leur caractère hydro- et/ou liposoluble), on peut citer notamment les épaississants ioniques ou non ioniques, les adoucissants, les antioxydants, les opacifiants, les stabilisants, les émollients, les agents répulsifs contre les insectes, les filtres solaires organiques actifs dans l'UV-A et/ou l'UV-B, les pigments et les nanopigments minéraux photoprotecteurs, les agents hydratants, les vitamines, les parfums, les conservateurs, les charges, les séquestrants, les colorants, ou tout autre ingrédient habituellement utilisé dans le domaine des produits autobronzants.

Bien entendu, l'homme de l'art veillera à choisir ce ou ces éventuels composés complémentaires et/ou leurs quantités de manière telle que les propriétés avantageuses attachées intrinsèquement à l'émulsion conforme à l'invention ne soient pas, ou substantiellement pas, altérées par la ou les adjonctions envisagées.

Un autre objet de la présente invention est constitué par l'utilisation des compositions telles que ci-dessus définies comme, ou pour la fabrication de, compositions cosmétiques pour le bronzage et/ou le brunissage artificiels de la peau. Comme indiqué précédemment, les compositions peuvent alors être conditionnées sous la forme de crèmes, de laits, de gels crèmes, ou bien encore de lotions fluides, en particulier de lotions fluides vaporisables, ou tout autre forme appropriée.

Un autre objet de la présente invention réside dans un procédé de traitement cosmétique de la peau destiné à la bronzer et/ou la brunir artificiellement et qui consiste essentiellement à appliquer sur celle-ci une quantité efficace d'une composition cosmétique telle que définie ci-dessus.

Des exemples concrets, mais nullement limitatifs, destinés à illustrer l'invention vont maintenant être donnés.

Dans tous les exemples qui suivent, les quantités sont exprimées en % en poids par rapport au poids total de la composition.

**EXEMPLE 1:**

La demanderesse a évalué au travers d'un test comparatif la coloration cutanée obtenue à partir de deux compositions contenant chacune 5% de DHA. Une première composition (1), à savoir une émulsion eau-dans-silicone conforme à l'invention et une deuxième composition (2), à savoir une émulsion H/E niosomée représentative de l'état de la technique, ont ainsi été réalisées.

Composition (1):

A:

| | |
|---|---|
| - polydiméthylsiloxane vendu sous la dénomination commerciale 〈〈 Silicone $Q_23225C$ 〉〉 par Dow Corning | 10 % |
| - polydiméthylsiloxane vendu sous la dénomination commerciale 〈〈 Silbione Huile 70 047 V 2 〉〉 par Rhône Poulenc | 7 % |

B:

| | |
|---|---|
| -dihydroxyacétone | 5 % |
| -dextrose anhydre | 2 % |
| -propylène glycol | 39 % |
| -eau déminéralisée | qsp 100% |

<u>Composition (2):</u>

A:

| *Vésicules lipidiques non ioniques (niosomes) dispersés*: | |
|---|---|
| - Alcool cétylique polyglycérolé à 3 moles de glycérol (Chimexane NL de Chimex) | 3,8 % |
| - Cholestérol | 3,8 % |
| - Stéaroyl Glutamate Monosodique (Acylglutamate HS 11 d'Ajinomoto) | 0,4 % |

D:

| *Phase huileuse liquide dispersée*: | |
|---|---|
| - Huile de vaseline | 8 % |
| - Cyclométhicone | 5 % |

B:

| | |
|---|---|
| *Eau*: | 8% |

E:

| *Phase aqueuse gélifiante*: | |
|---|---|
| - Hydroxyéthylcellulose | 0,5 % |
| - Conservateurs | qs |
| - Eau | 30 % |

C:

| *Phase aqueuse dispersante* : | |
|---|---|
| - Glycérine | 2 % |
| - Dihydroxyacétone | 5 % |
| - Eau | qsp 100% |

La composition (1) a été réalisée de la manière suivante: à température ambiante, on a versé lentement B dans A sous agitation Moritz douce à 1500 t/min. Puis on a laissé la composition sous agitation pendant 10 min environ en augmentant progressivement la vitesse jusqu'à environ 4500 t/min.

La composition (2) a quant à elle été préparée de la manière suivante: on a préparé la phase E par dispersion du gélifiant dans l'eau à 60°C sous agitation. On a ensuite chauffé la phase A et la phase B séparément à 80-85 °C jusqu'à homogénéisation. Puis on a ajouté B à A sous forte agitation Turrax. Lorsque le mélange (A+B) est revenu à température ambiante, on a ajouté la phase C puis la phase D. On a finalement ajouté la phase E sous agitation douce.

Pour ces deux compositions ainsi préparées, on a ensuite déterminé l'intensité de coloration qui leur était attachée après un certain temps T, soit $\Delta L_T$. La mesure de l'intensité de coloration a été effectuée selon la méthode suivante: on a appliqué ces formulations sur des zones de 4,5 cm x 4,5 cm, à raison de 2mg/cm$^2$ de peau, sur le dos de 5 modèles humains, puis on a effectué des mesures colorimétriques à l'aide d'un colorimètre Minolta CR 200, 1H30, 3H, 5H et 24H après l'application des produits.

Les résultats en ΔL moyens sont rassemblés dans le tableau (I) ci-dessous:

Tableau (I)

| | composition (1) | composition (2) |
|---|---|---|
| ΔL 1H30 | 1,2 | 0,3 |
| ΔL 3H | 1,9 | 1,1 |
| ΔL 5H | 3,2 | 2,4 |
| ΔL 24H | 4,2 | 3,2 |

Ces résultats montrent clairement que l'on obtient une montée de coloration plus rapide et significativement plus intense avec la composition conforme à l'invention.

**<u>EXEMPLE 2</u>:**

Cet exemple a pour but de montrer le caractère critique de l'émulsionnant siliconé conforme à l'invention. Deux compositions, une composition (3) conforme à l'invention et une composition (4) comparative ne contenant pas l'émulsionnant siliconé tel que défini dans la présente invention mais un autre émulsionnant siliconé, ont été réalisées selon le mode opératoire utilisé pour la composition (1) de l'exemple 1.

Composition (3):

| | |
|---|---|
| - polydiméthylsiloxane vendu sous la dénomination commerciale ⟨⟨ Silicone $Q_2$3225C ⟩⟩ par Dow Corning | 10 % |
| - polydiméthylsiloxane vendu sous la dénomination commerciale ⟨⟨ Silbione Huile 70 047 V 2 ⟩⟩ par Rhône Poulenc | 7 % |
| -dihydroxyacétone | 5 % |
| -dextrose anhydre | 2 % |
| -propylène glycol | 20 % |
| -conservateurs | qs |
| -eau déminéralisée | qsp 100% |

Composition (4):

| | |
|---|---|
| - cétyl diméthicone copolyol vendue sous la dénomination commerciale ⟨⟨ Silicone Abil EM 90 ⟩⟩ par Goldschmidt | 1 % |
| - polydiméthylsiloxane vendu sous la dénomination commerciale ⟨⟨ Silbione Huile 70 047 V 2 ⟩⟩ par Rhône Poulenc | 16 % |
| -dihydroxyacétone | 5 % |
| -dextrose anhydre | 2 % |
| -propylène glycol | 20 % |
| -conservateurs | qs |
| -eau déminéralisée | qsp 100% |

Pour ces deux compositions ainsi préparées, la mesure de l'intensité de coloration a été effectuée selon la méthode suivante: on a appliqué ces formulations sur des zones de 2,5 cm x 2,5 cm, à raison de 2mg/$cm^2$ de peau, sur la face interne des avant-bras de 3 modèles humains, à savoir $M_1$, $M_2$ et $M_3$ puis on a effectué des mesures colorimétriques à l'aide d'un colorimètre Minolta CM 1000, 3H après l'application des produits. Les résultats sont consignés dans le tableau (II) ci-dessous:

Tableau (II)

| $\Delta$L 3H | **Composition (3)** | **Composition (4)** |
|---|---|---|
| $M_1$ | 3,32 | 2,34 |
| $M_2$ | 4,09 | 2,92 |
| $M_3$ | 3,53 | 2,98 |
| **$\Delta$L** | **3,65** | **2,75** |

Ces résultats montrent clairement qu'une coloration plus intense est observée avec la composition contenant l'émulsionnant siliconé conforme à l'invention.

**EXEMPLE 3**:

Cet exemple a pour but de montrer le caractère critique de l'alcool conforme à l'invention.

Deux compositions, une composition (5) conforme à l'invention et une composition (6) comparative ne contenant pas d'alcool tel que défini dans la présente invention, ont été réalisées selon le mode opératoire utilisé pour la composition (1) de l'exemple 1.

Composition (5):

| | |
|---|---|
| - polydiméthylsiloxane vendu sous la dénomination commerciale ⟨⟨ Silicone $Q_2$3225C ⟩⟩ par Dow Corning | 10 % |
| - polydiméthylsiloxane vendu sous la dénomination commerciale ⟨⟨ Silbione Huile 70 047 V 2 ⟩⟩ par Rhône Poulenc | 7 % |
| -dihydroxyacétone | 5 % |
| -dextrose anhydre | 2 % |
| -propylène glycol | 30 % |
| -eau déminéralisée | qsp 100% |

Composition (6):

| | |
|---|---|
| - polydiméthylsiloxane vendu sous la dénomination commerciale ⟨⟨ Silicone $Q_2$3225C ⟩⟩ par Dow Corning | 10 % |
| - polydiméthylsiloxane vendu sous la dénomination commerciale ⟨⟨ Silbione Huile 70 047 V 2 ⟩⟩ par Rhône Poulenc | 7 % |
| -dihydroxyacétone | 5 % |
| -dextrose anhydre | 2 % |
| -glycérine | 30 % |
| -eau déminéralisée | qsp 100% |

Pour ces deux compositions, le test comparatif a été réalisé selon le même protocole que pour l'exemple 2. Les résultats sont consignés dans le tableau (III) ci-dessous:

Tableau (III)

| ΔL 3H | **Composition (5)** | **Composition (6)** |
|---|---|---|
| $M_1$ | 4,34 | 2,03 |
| $M_2$ | 5,04 | 3,55 |
| $M_3$ | 4,46 | 3,84 |
| **ΔL** | **4,63** | **2,80** |

Ces résultats montrent clairement qu'une coloration plus intense est obtenue avec la composition contenant le propylène glycol conforme à l'invention.

**Revendications**

1. Composition cosmétique destinée au bronzage artificiel de la peau du type comprenant, dans un support cosmétiquement acceptable de type eau-dans-silicone, de la DHA à titre d'agent autobronzant, caractérisée par le fait qu'elle contient en outre:

   - au moins un alcool mono ou dihydrique,
   - au moins un émulsionnant siliconé constitué d'un polydiméthylsiloxane de formule (I) ou (II) suivante:

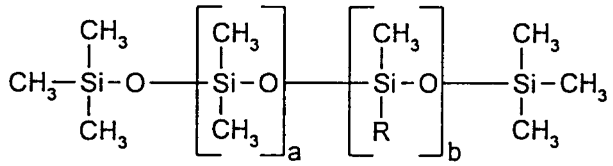

(I)

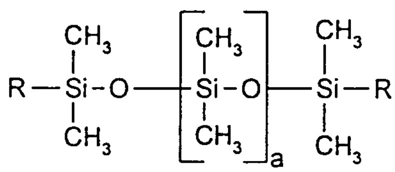

(II)

   formules (I) et (II) dans lesquelles:

   - $1 \leq a \leq 500$ (a étant un nombre entier)
   - $1 \leq b \leq 100$ (b étant un nombre entier)
   - R représente un radical:

$$—C_nH_{2n}—(—OC_2H_4)_x—(—OC_3H_6)_y—O—R'$$

   dans lequel R' représente H ou un radical alkyle linéaire ou ramifié en $C_1$-$C_{12}$ et:

   - $0 \leq x \leq 50$ (x étant un nombre entier)
   - $0 \leq y \leq 50$ (y étant un nombre entier)
   - $x + y \geq 1$
   - $2 \leq n \leq 12$ (n étant un nombre entier)

2. Composition cosmétique selon la revendication 1, caractérisée en ce que l'émulsionnant siliconé de formule (I) ou (II) présente au moins l'une des caractéristiques suivantes:

- $2 \leq a \leq 450$
- $2 \leq b \leq 40$
- $1 \leq x \leq 30$
- $0 \leq y \leq 30$
- $y \leq x$

3. Composition cosmétique selon la revendication 2, caractérisée en ce que ledit émulsionnant siliconé présente l'ensemble desdites caractéristiques.

4. Composition cosmétique selon l'une quelconque des revendications précédentes, caractérisée en ce que R' représente l'hydrogène.

5. Composition cosmétique selon la revendication 4, caractérisée en ce que l'émulsionnant siliconé est le polydiméthylsiloxane de formule suivante:

$$Me_3SiO\text{-}(Me_2SiO)_{396}\text{-}(MeSiO)_4\text{-}SiMe_3$$
$$|$$
$$(CH_2)_3\text{-}EO_{18}\text{-}PO_{18}\text{-}OH$$

6. Composition cosmétique selon l'une quelconque des revendications précédentes, caractérisée en ce que l'émulsionnant siliconé est un polydiméthylsiloxane de formule (I) ou (II) dispersé dans un polydiméthylsiloxane volatil.

7. Composition cosmétique selon l'une quelconque des revendications précédentes, caractérisée en ce que ledit alcool est un alcool dihydrique.

8. Composition cosmétique selon la revendication 7, caractérisée en ce que ledit alcool dihydrique est le propylène glycol.

9. Composition cosmétique selon l'une quelconque des revendications précédentes, caractérisée en ce que l'alcool mono ou dihydrique représente au moins 2% en poids du poids total de la composition.

10. Composition cosmétique selon l'une quelconque des revendications précédentes, caractérisée en ce que l'alcool mono ou dihydrique représente entre 10 et 50% en poids du poids total de la composition.

11. Composition cosmétique selon l'une quelconque des revendications précédentes, caractérisée en ce que l'émulsionnant siliconé représente entre 0,1 et 20% en poids du poids total de la composition.

12. Composition cosmétique selon l'une quelconque des revendications précédentes, caractérisée en ce que l'émulsionnant siliconé représente entre 0,5 et 10% en poids du poids total de la composition.

13. Utilisation d'une composition cosmétique telle que définie dans l'une quelconque des revendications précédentes comme ou pour la fabrication de compositions destinées au bronzage et/ou au brunissage artificiels de la peau.

14. Procédé de traitement cosmétique de la peau destiné à la bronzer et/ou la brunir artificiellement, caractérisé en ce qu'il consiste à appliquer sur celle-ci une quantité efficace d'une composition cosmétique telle que définie à l'une quelconque des revendications 1 à 12.

## Claims

1. Cosmetic composition intended for the artificial tanning of the skin, of the type comprising, in a cosmetically acceptable support of water-in-silicone type, DHA as self-tanning agent, characterized in that it also contains:

- at least one mono- or dihydric alcohol,
- at least one silicone-containing emulsifying agent consisting of a polydimethylsiloxane of formula (I) or (II) below,

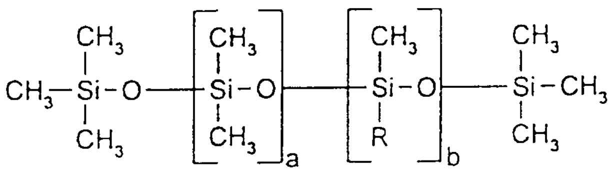

(I)

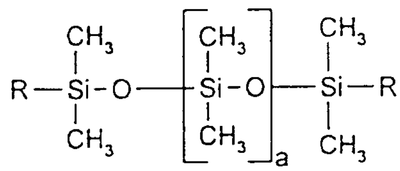

(II)

in which formulae (I) and (II):

- $1 \leq a \leq 500$ (a being an integer)
- $1 \leq b \leq 100$ (b being an integer)
- R represents a radical:

$$-C_nH_{2n}-(-OC_2H_4)_x-(-OC_3H_6)_y-O-R'$$

in which R' represents H or a linear or branched $C_1$-$C_{12}$ alkyl radical, and:

- $0 \leq x \leq 50$ (x being an integer)
- $0 \leq y \leq 50$ (y being an integer)
- $x + y \geq 1$
- $2 \leq n \leq 12$ (n being an integer)

2. Cosmetic composition according to Claim 1, characterized in that the silicone-containing emulsifying agent of formula (I) or (II) has at least one of the following characteristics:

- $2 \leq a \leq 450$
- $2 \leq b \leq 40$
- $1 \leq x \leq 30$
- $0 \leq y \leq 30$
- $y \leq x$

3. Cosmetic composition according to Claim 2, characterized in that the said silicone-containing emulsifying agent has all of the said characteristics.

4. Cosmetic composition according to any one of the preceding claims, characterized in that R' represents hydrogen.

5. Cosmetic composition according to Claim 4, characterized in that the silicone-containing emulsifying agent is the polydimethylsiloxane of the following formula:

$$Me_3SiO\text{-}(Me_2SiO)_{396}\text{-}(MeSiO)_4\text{-}SiMe_3$$

$$|$$

$$(CH_2)_3\text{-}EO_{18}\text{-}PO_{18}\text{-}OH$$

6. Cosmetic composition according to any one of the preceding claims, characterized in that the silicone-containing emulsifying agent is a polydimethylsiloxane of formula (I) or (II) dispersed in a volatile polydimethylsiloxane.

7. Cosmetic composition according to any one of the preceding claims, characterized in that the said alcohol is a dihydric alcohol.

8. Cosmetic composition according to Claim 7, characterized in that the said dihydric alcohol is propylene glycol.

9. Cosmetic composition according to any one of the preceding claims, characterized in that the mono- or dihydric alcohol represents at least 2 % by weight of the total weight of the composition.

10. Cosmetic composition according to any one of the preceding claims, characterized in that the mono- or dihydric alcohol represents between 10 and 50 % by weight of the total weight of the composition.

11. Cosmetic composition according to any one of the preceding claims, characterized in that the silicone-containing emulsifying agent represents between 0.1 and 20 % by weight of the total weight of the composition.

12. Cosmetic composition according to any one of the preceding claims, characterized in that the silicone-containing emulsifying agent represents between 0.5 and 10 % by weight of the total weight of the composition.

13. Use of a cosmetic composition as defined in any one of the preceding claims as, or for the manufacture of, compositions intended for the artificial tanning and/or bronzing of the skin.

14. Process for the cosmetic treatment of the skin which is intended to artificially tan and/or bronze it, characterized in that it consists in applying an effective amount of a cosmetic composition as defined in any one of Claims 1 to 12, to the skin.

**Patentansprüche**

1. Kosmetische Zusammensetzungen, die zur künstlichen Bräunung der Haut bestimmt sind und die in einem kosmetisch akzeptablen Träger vom Typ Wasser-in-Silicon DHA als Selbstbräunungsmittel enthalten,
dadurch gekennzeichnet, daß sie ferner enthalten:

- mindestens einen einwertigen oder zweiwertigen Alkohol,

- mindestens einen Siliconemulgator, der aus einem Polydimethylsiloxan der folgenden Formel I oder II besteht

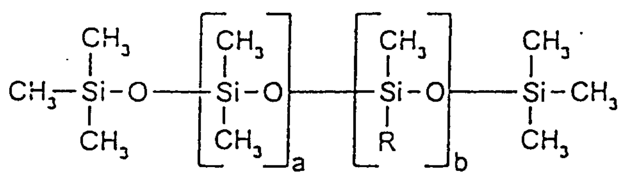 (I),

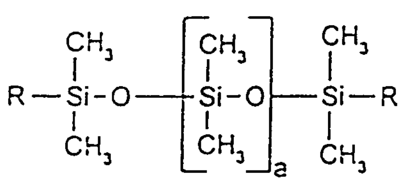 (II),

wobei in den Formeln I und II bedeuten

- $1 \leq a \leq 500$ (wobei a eine ganze Zahl bedeutet),
- $1 \leq b \leq 100$ (wobei b eine ganze Zahl bedeutet),
- R eine Gruppe

$$—C_nH_{2n}—(—OC_2H_4)_x—(—OC_3H_6)_y—O—R'$$

worin bedeuten:

- R' H oder eine geradkettige oder verzweigte $C_{1-12}$-Alkylgruppe,
- $0 \leq x \leq 50$ (wobei x eine ganze Zahl bedeutet),
- $0 \leq y \leq 50$ (wobei y eine ganze Zahl bedeutet),
- $x + y \geq 1$,
- $2 \leq n \leq 12$ (wobei n eine ganze Zahl bedeutet).

**2.** Kosmetische Zusammensetzung nach Anspruch 1, dadurch gekennzeichnet, daß der Siliconemulgator der Formel I oder II mindestens eine der folgenden Eigenschaften aufweist

- $2 \leq a \leq 450$
- $2 \leq b \leq 40$
- $1 \leq x \leq 30$
- $0 \leq y \leq 30$
- $y \leq x$.

**3.** Kosmetische Zusammensetzung nach Anspruch 2, dadurch gekennzeichnet, daß der Siliconemulgator alle angegebenen Eigenschaften aufweist.

**4.** Kosmetische Zusammensetzung nach einem der vorhergehenden Ansprüche, dadurch gekennzeichnet, daß R' Wasserstoff bedeutet.

**5.** Kosmetische Zusammensetzung nach Anspruch 4, dadurch gekennzeichnet, daß der Siliconemulgator das Polydimethylsiloxan der folgenden Formel ist:

$$Me_3SiO-(Me_2SiO)_{396}-(MeSiO)_4-SiMe_3$$
$$|$$
$$(CH_2)_3-EO_{18}-PO_{18}-OH$$

**6.** Kosmetische Zusammensetzung nach einem der vorhergehenden Ansprüche, dadurch gekennzeichnet, daß der Siliconemulgator ein Polydimethylsiloxan der Formel I oder II ist und in einem flüchtigen Polydimethylsiloxan dispergiert ist.

**7.** Kosmetische Zusammensetzung nach einem der vorhergehenden Ansprüche, dadurch gekennzeichnet, daß der Alkohol ein zweiwertiger Alkohol ist.

**8.** Kosmetische Zusammensetzung nach Anspruch 7, dadurch gekennzeichnet, daß der zweiwertige Alkohol Propylenglykol ist.

**9.** Kosmetische Zusammensetzung nach einem der vorhergehenden Ansprüche, dadurch gekennzeichnet, daß der ein- oder zweiwertige Alkohol mindestens 2 Gew.-% des Gesamtgewichts der Zusammensetzung ausmacht.

**10.** Kosmetische Zusammensetzung nach einem der vorhergehenden Ansprüche, dadurch gekennzeichnet, daß der einoder zweiwertige Alkohol 10 bis 50 Gew.-% des Gesamtgewichts der Zusammensetzung ausmacht.

**11.** Kosmetische Zusammensetzung nach einem der vorhergehenden Ansprüche, dadurch gekennzeichnet, daß der Siliconemulgator 0,1 bis 20 Gew.-% des Gesamtgewichts der Zusammensetzung ausmacht.

**12.** Kosmetische Zusammensetzung nach einem der vorhergehenden Ansprüche, dadurch gekennzeichent, daß der Siliconemulgator 0,5 bis 10 Gew.-% des Gesamtgewichts der Zusammensetzung ausmacht.

**13.** Verwendung der kosmetischen Zusammensetzungen nach einem der vorhergehenden Ansprüche als Zusammensetzungen, die zur künstlichen Bräunung und/oder Braunfärbung der Haut bestimmt sind, oder zur Herstellung dieser Zusammensetzungen.

**14.** Verfahren zur kosmetischen Behandlung der Haut zur künstlichen Bräunung und/oder Braunfärbung, dadurch gekennzeichnet, daß es darin besteht, auf die Haut eine wirksame Menge einer kosmetischen Zusammensetzung nach einem der Ansprüche 1 bis 12 aufzutragen.